(19) Europäisches Patentamt
European Patent Office
Office européen des brevets

(11) **EP 1 902 750 B1**

(12) **FASCICULE DE BREVET EUROPEEN**

(45) Date de publication et mention
de la délivrance du brevet:
**19.11.2008 Bulletin 2008/47**

(21) Numéro de dépôt: **07291085.4**

(22) Date de dépôt: **11.09.2007**

(51) Int Cl.:
*A61N 1/37* (2006.01)     *A61B 5/04* (2006.01)
*A61B 5/0402* (2006.01)     *G06F 17/00* (2006.01)
*A61N 1/372* (2006.01)

(54) **Procédé de reconstruction d'un électrocardiogramme de surface à partir d'un électrogramme endocavitaire**

Verfahren zur Rekonstruktion eines Oberflächenelektrokardiogramms aus einer endokavitären Aktionsstromkurve

Method of reconstructing a surface electrocardiogram from an intracardiac electrogram

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **25.09.2006 FR 0608367**

(43) Date de publication de la demande:
**26.03.2008 Bulletin 2008/13**

(73) Titulaire: **ELA MEDICAL**
**92541 Montrouge (FR)**

(72) Inventeurs:
- **Bourguiba, Anissa**
**75005 Paris (FR)**
- **Dal Molin, Renzo**
**92320 Châtillon (FR)**
- **Carrault, Guy**
**35510 Cesson-Sévigné (FR)**
- **Hernandez, Alfredo**
**35700 Rennes (FR)**

- **Porée, Fabienne**
**35000 Rennes (FR)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**US-A- 5 740 811      US-A1- 2003 083 587**
**US-A1- 2005 197 674      US-B1- 6 980 850**

- **SCHERER J A ET AL: "SYNTHESIS OF THE 12 LEAD ELECTROCARDIOGRAM FROM A 3 LEAD SEMI-ORTHOGONAL SUBSET USING PATIENT-SPECIFIC LINEAR TRANSFORMATION ARRAYS" PROCEEDINGS OF THE COMPUTERS IN CARDIOLOGY MEETING. WASHINGTON, SEPT. 25 - 28, 1988, WASHINGTON, IEEE COMP. SOC. PRESS, US, vol. MEETING 15, janvier 1989 (1989-01), pages 449-451, XP002004856 ISBN: 0-8186-1949-X**

EP 1 902 750 B1

**Description**

L'invention a trait au domaine des "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resyn-chronisation, de cardioversion et/ou de défibrillation en cas de trouble du rythme détecté par le dispositif.

L'invention concerne plus particulièrement le traitement des signaux représentatifs des potentiels cardiaques de dépolarisation du myocarde, signaux recueillis par des électrodes épicardiques ou endocavitaires de stimulation ou de détection ou de défibrillation des oreillettes ou des ventricules droits et gauches de ces dispositifs implantés.

Plus précisément, l'invention propose un dispositif permettant de reconstruire un électrocardiogramme de surface (ECG) à partir d'un électrogramme endocavitaire ou épicardique (EGM).

Les signaux EGM sont des signaux recueillis par des électrodes placées sur les sondes endocavitaires ou épicardiques implantées avec le dispositif. Ces signaux; directement issus de l'activité électrique des cellules cardiaques, fournissent une information très riche pour évaluer l'état du patient. De fait, après amplification, numérisation et filtrage, ils sont principalement utilisés pour piloter le stimulateur cardiaque et pour diagnostiquer certains troubles du rythme nécessitant le déclenchement automatique d'une thérapie antitachycardique, antibradycardique ou de resynchronisation interventriculaire, par mise en oeuvre d'algorithmes élaborés d'analyse et de prise de décision.

En revanche, lorsqu'il s'agit d'analyser de façon subjective le rythme pour poser un diagnostic ou pour réajuster les paramètres d'un implant, les médecins préfèrent, en pratique, interpréter les informations d'électrocardiogramme de surface (ECG), qui permettent de visualiser de façon directe un certain nombre de facteurs importants (largeur du QRS, ...) et apprécier l'évolution d'une insuffisance cardiaque.

En effet, les signaux ECG et EGM, bien qu'ils aient la même source (l'activité électrique du myocarde), se présentent visuellement de façon assez différente : l'EGM recueilli par la prothèse cardiaque fournit une information locale sur l'activité électrique d'un groupe de cellules cardiaques, tandis que l'ECG apparaît sous la forme d'une information plus globale, influencée par la propagation du signal électrique entre le myocarde et la surface du corps, et par un certain nombre de différences morphologiques et pathologiques. Ainsi, l'affichage des signaux EGM n'est pas parlant pour un médecin habitué à interpréter de longue date des ECG de surface.

Ce sont également les signaux ECG qui sont enregistrés sur une longue période en ambulatoire par les dispositifs Holter, pour être ensuite traités et analysés afin d'évaluer l'état clinique du patient et diagnostiquer le cas échéant un trouble du rythme cardiaque.

De ce fait, lorsqu'un patient porteur d'un implant vient voir son médecin pour une visite de contrôle, celui-ci utilise en pratique deux appareils, à savoir un enregistreur ECG et un programmateur d'implant. Pour recueillir l'ECG, le praticien colle un certain nombre d'électrodes sur le torse du patient, de manière à définir les douze dérivations usuelles correspondant à autant de signaux ECG différents. Le programmateur externe est, quant à lui, utilisé pour contrôler certains paramètres de fonctionnement de l'implant (par exemple la durée de vie de la pile), télécharger des données enregistrées dans la mémoire de l'implant, et éventuellement modifier le paramétrage de ce dernier, y télécharger une version modifiée du logiciel de commande, etc.

La consultation chez le médecin requiert donc généralement deux appareils différents, ainsi qu'une manipulation pour la pose des électrodes de surface et le recueil des signaux ECG.

Le recours à ces deux appareils implique de plus le déplacement du patient dans un centre spécialement équipé, avec pour conséquence le plus souvent un espacement des visites de contrôle et donc un moindre suivi du patient.

Pour remédier à ces inconvénients, on a cherché à développer des algorithmes de reconstruction de l'ECG de surface à partir des signaux EGM, c'est-à-dire à partir des signaux directement délivrés par la prothèse implantée. Une reconstruction de l'ECG de surface à partir des signaux EGM permettrait en effet :

- d'éviter, lors de la consultation, d'avoir à poser des électrodes et à recourir à un enregistreur ECG ;
- de rendre ainsi la consultation plus simple et plus rapide, éventuellement pouvoir la faire à domicile et par voie de conséquence rapprocher les contrôles et améliorer le suivi du patient ;
- et d'autoriser éventuellement une télétransmission des données EGM enregistrées par la prothèse, sans intervention d'un praticien ou auxiliaire médical auprès du patient.

Divers algorithmes de reconstruction de l'ECG de surface à partir des signaux EGM ont été proposés.

Le US-A-5 740 811 (Hedberg et al.) prévoit de synthétiser un signal ECG de surface par combinaison d'une pluralité de signaux EGM au moyen d'un réseau de neurones et/ou d'une logique floue et/ou d'un circuit sommateur, après apprentissage effectué par un algorithme du type *feedforward*.

Cette technique, qui opère par un filtrage monodimensionnel linéaire, présente l'inconvénient de produire en sortie un signal qui ne correspond qu'à une seule dérivation de l'ECG de surface et n'offre donc au praticien qu'une vision très réduite de l'activité cardiaque du patient, par rapport aux douze dérivations habituelles délivrées par un enregistreur ECG externe. Un autre inconvénient de cette technique est qu'elle ne tient pas compte de la position des sondes endocavitaires, qui peut changer entre le moment de l'apprentissage et celui de l'utilisation du dispositif : une modification de l'axe électrique du coeur aura pour effet de biaiser le signal ECG synthétisé, qui ne sera plus significatif, avec le risque de masquer un trouble du rythme qui ne sera pas diagnostiqué.

Le US-A-6 980 850 (Kroll et al.) tente de remédier à cette difficulté, en proposant une méthode de reconstruction de l'ECG de surface mettant en oeuvre une transformation matricielle permettant de restituer individuellement chacune des dérivations de l'ECG de surface. Cette transformation permet en outre de prendre en compte plusieurs paramètres, tels que la respiration ou la posture du patient, qui influencent la localisation des sondes intracardiaques dans l'espace. La reconstruction décrite consiste à transformer, par une matrice de transfert prédéterminée, un vecteur d'entrée représentatif d'une pluralité de signaux EGM en un vecteur résultant représentatif des différentes dérivations ECG. La matrice de transfert est apprise par moyennage de plusieurs matrices instantanées à partir de vecteurs ECG et EGM enregistrés simultanément sur une même durée au cours d'une phase d'apprentissage.

Cette technique, si elle constitue un progrès par rapport à celle du brevet précédent, présente cependant un certain nombre d'inconvénients. En premier lieu, elle suppose qu'il existe une relation linéaire entre les vecteurs ECG et EGM : cette approximation, si elle se révèle satisfaisante avec des patients présentant un rythme régulier, entraîne dans un certain nombre de cas des erreurs importantes de reconstruction de l'ECG en présence de morphologies de signal atypiques ou irrégulières - correspondant précisément à des situations potentiellement pathologiques. D'autre part, les paramètres de la matrice de transfert sont déterminés au cours d'une phase d'apprentissage correspondant à un état du patient à un instant donné, situation qui ne sera peut-être plus représentative plusieurs semaines ou mois après, compte tenu notamment de l'évolution de la pathologie de ce patient ; cette évolution ne sera pas prise en compte par l'algorithme, sauf à exiger du patient qu'il revienne dans un service clinique pour recalibration de l'algorithme (calcul d'une nouvelle matrice de transfert).

D'autres approches ont été envisagées, par exemple celle décrite dans le US-A-2005/0288600 (Zhang et al. ), qui consiste à utiliser non pas des signaux EGM d'électrogramme (qui requièrent l'utilisation d'électrodes placées sur des sondes endocavitaires), mais des signaux d'ECG subcutané recueillis à partir d'un nombre réduit d'électrodes directement placées sur le boîtier du générateur implanté. L'ECG est alors obtenu directement depuis l'intérieur du corps du patient au lieu de l'être par des électrodes de surface appliquées sur la peau, comme dans le cas des enregistreurs ECG traditionnels. Les différents signaux d'ECG subcutané recueillis sont séparés et soumis à une analyse (morphologie, intervalles temporels, analyse fréquentielle, ...) en fonction de critères stockés dans une mémoire. Le résultat de cette analyse est comparé à une référence mémorisée au préalable et mise à jour par le système, notamment lorsque des changements ont lieu. L'analyse des signaux permet de suivre l'évolution du rythme cardiaque du patient pour faire un diagnostic cardiaque.

Cependant, déposer des électrodes sur un boîtier est technologiquement difficile à réaliser.

L'invention a pour but de remédier à ces différents inconvénients, en proposant un dispositif pourvu de moyens de reconstruction d'un ECG de surface se présentant avec l'ensemble de ses dérivations telles qu'elles peuvent être présentées à un praticien lorsque celui-ci utilise un enregistreur traditionnel, et qui tienne compte des différentes contraintes suivantes :

- paramètres propres au patient : orientation et taille du coeur, épaisseurs des tissus biologiques, ...
- nature et évolution de la pathologie,
- type de prothèse implantée (stimulateur, défibrillateur ou resynchroniseur, ou encore dispositif double, triple ou quadruple chambre, etc ...),
- localisation des sondes endocavitaires.

Ceci avec les avantages suivants :

- procédé partiellement et totalement indépendant du patient,
- ECG reconstruit proche de l'ECG réel,
- procédé ne nécessitant pas une puissance de calcul trop importante,
- mise en oeuvre possible aussi bien dans l'implant, que dans un programmateur externe, un moniteur de *home monitoring* ou un serveur de données distant, à partir d'informations télétransmises à ce dernier.

Essentiellement, l'invention est basée sur une approche vectorielle consistant à estimer l'ECG de surface non pas directement à partir de l'EGM endocavitaire, mais en utilisant comme intermédiaire une représentation vectorielle tridimensionnelle des divers signaux ECG ou EGM, respectivement.

L'invention propose un dispositif du type générique tel que celui décrit par le US-A-6 980 850 précité, comprenant des moyens aptes à exécuter les étapes sucessives énoncées à la revendication 1. Des formes de réalisation préférentielles sont présentées dans les sous-revendications.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 illustre, en projection sur trois plans et en perspective axonométrique, la boucle décrite par un vectocardiogramme VCG typique au cours d'un cycle cardiaque.

La figure 2 illustre de façon schématique les étapes effectuées par le dispositif selon l'invention, permettant la reconstruction d'un électrocardiogramme ECG à partir des signaux d'électrogramme EGM produits par un implant.

La figure 3 illustre, en projection sur trois plans et en perspective axonométrique, la boucle décrite par un vectogramme VGM typique au cours de trois cycles cardiaques successifs.

La figure 4 illustre de façon schématique les différentes étapes mises en oeuvre au cours de la phase préalable de calibration, pour définir les paramètres du recalage angulaire à appliquer au VGM.

La figure 5 illustre de façon schématique les différentes étapes mises en oeuvre au cours de la phase préalable de calibration, pour définir les paramètres du filtrage vectoriel non linéaire permettant de reconstruire le VCG à partir du VGM recalé.

La figure 6 est un exemple comparatif montrant l'allure de l'ECG reconstruit à partir des signaux EGM par le dispositif de l'invention, par rapport à un ECG réel recueilli au moyen d'électrodes de surface suivant une technique conventionnelle.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires.

L'invention peut notamment être appliquée aux dispositifs implantables commercialisés par ELA Médical, Montrouge, France tels que les appareils *Symphony* et *ELA Rhapsody.* Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le dispositif de l'invention peut cependant être mise en oeuvre non seulement au sein d'un implant (traitement direct des signaux VGM), mais également dans un programmateur externe utilisé par un praticien de manière à télécharger et analyser les signaux cardiaques recueillis et mémorisés par un implant. Dans une variante avantageuse, le dispositif de l'invention est également mis en oeuvre dans un moniteur de *home monitoring,* qui est un type particulier de programmateur dont le fonctionnement est entièrement automatisé et ne nécessite pas le recours à un praticien, notamment pour permettre à intervalles réguliers, par exemple quotidiennement, la télétransmission à un site distant des données recueillies, pour analyse et suivi du patient. Le dispositif de l'invention peut également être mis en oeuvre au niveau du serveur de données de ce site, les données EGM brutes étant alors télétransmises directement à ce serveur, sans traitement préalable. Le traitement peut être effectué alors par le serveur ou le terminal (PC ou programmateur) connecté à ce dernier.

De façon générale, l'activité électrique cardiaque se manifeste à la surface du corps du patient par des signaux dits électrocardiographiques (ECG), qui sont recueillis entre des paires d'électrodes appliquées en des endroits prédéterminés du thorax du patient, chaque paire d'électrodes déterminant une dérivation. Le tout forme un ensemble de douze dérivations, de sorte que l'activité électrique cardiaque peut être assimilée à un vecteur en douze dimensions variant au cours du temps. Les dérivations bipolaires (I, II, III) et unipolaires (aVF, aVR, aVL) permettent de représenter l'activité électrique dans le plan frontal, alors que les précordiales (v1 à v6) la représentent dans le plan horizontal.

L'activité électrique de surface peut être également représentée sous forme graphique tridimensionnelle par un vecteur en coordonnées x, y, z, comme cela a été exposé par E. Frank, An Accurate, Clinically Practical System for Spatial Vectocardiography, Circulation, 13:737-749, mai 1956. Cette représentation, appelée vectocardiogramme (VCG), peut être obtenue à partir d'un ensemble de sept électrodes de surface, et elle contient toute l'information sur les processus de dépolarisation et repolarisation du myocarde, c'est-à-dire qu'elle est aussi riche, du point de vue de l'information, que les douze signaux ECG unidimensionnels.

Le VCG se présente ainsi sous forme d'un vecteur dont le module et la direction (par rapport au repère défini par le thorax du patient) varient de façon constante au cours du temps. L'extrémité de ce vecteur, à chaque battement cardiaque, décrit une boucle représentant visuellement l'activité cardiaque du patient.

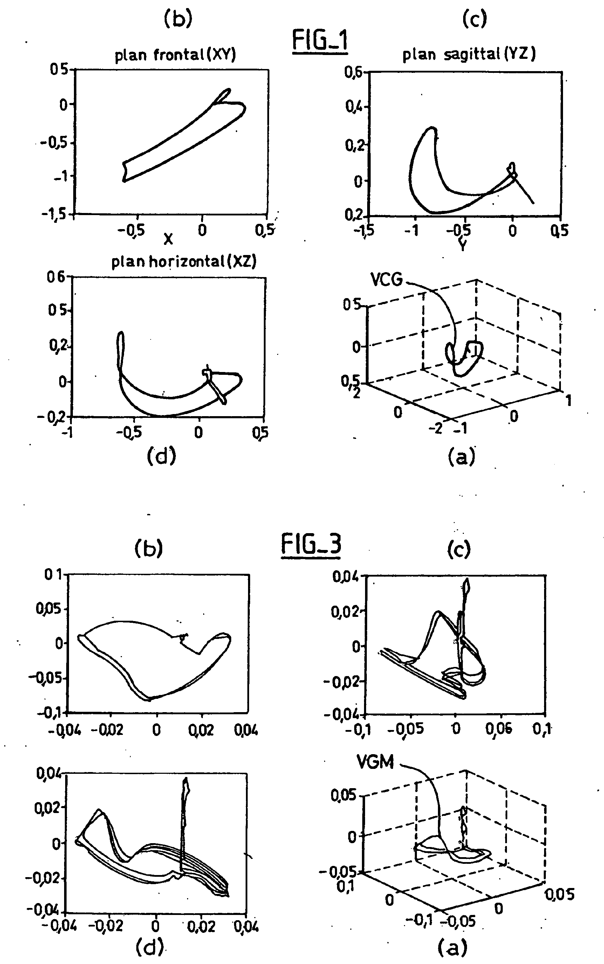

La figure 1 illustre un exemple d'une telle boucle décrite par l'extrémité du vecteur VCG au cours d'un cycle cardiaque. Sur cette figure, la boucle VCG est représentée en perspective (figure 1a) et en projection sur les trois plans frontal, horizontal et sagittal (figures 1b à 1d).

Il a par ailleurs été démontré, comme cela est exposé notamment dans le US-A-4 850 370 (Dower), qu'il est possible de réduire le système de Frank, qui comprenait sept électrodes, à un système à quatre électrodes seulement, dit système EASI. Il a également été défini deux matrices de transformation, dites "matrices de Dower" et "matrice de Dower inverse", permettant de retrouver les douze dérivations ECG (signaux unidimensionnels) à partir du VCG, et inversement. En d'autres termes, ce document décrit une transformation biunivoque entre la pluralité (douze) de signaux ECG définis uniquement dans le domaine temporel, et les variations dans ce même domaine temporel de la représentation graphique tridimensionnelle et vectorielle du vectocardiogramme VCG.

L'idée de base de l'invention consiste à reconstruire les signaux des dérivations ECG de surface à partir des signaux EGM endocavitaires recueillis par la prothèse implantée, via une transformation vectorielle intermédiaire impliquant une reconstruction du VCG.

Essentiellement, le dispositif de l'invention comprend des moyens aptes à exécuter les étapes suivantes :

- recueillir des signaux endocavitaires d'électrogramme (EGM),
- construire le vectogramme (VGM) correspondant,
- transformer ce vectogramme (VGM) en vectocardiogramme (VCG),
- reconstituer les signaux d'électrocardiogramme (ECG) de surface à partir du VCG au moyen d'une matrice de Dower inverse (ou toute autre technique procurant le même résultat).

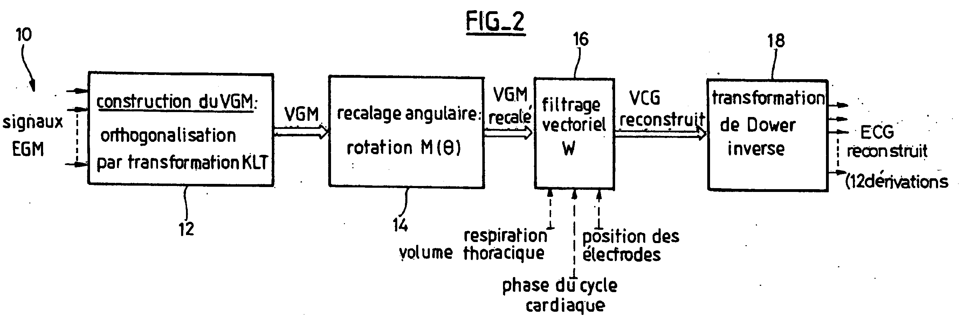

Ces différentes étapes successives sont illustrées par le schéma de la figure 2, avec les étapes successives suivantes :

- Étape 10 : sélection de dérivations EGM de l'implant permettant de recueillir les signaux EGM qui permettront de construire un vectogramme endocavitaire correspondant,
- Étape 12 : construction du vectogramme endocavitaire *VGM,* représentation tridimensionnelle du vecteur cardiaque à partir des signaux EGM d'électrogramme. Le vectogramme *VGM* a trois composantes orthogonales donnant la propagation de l'activité cardiaque dans trois plans x, y, z et ses composantes sont calculées par une orthogonalisation en s'appuyant par exemple sur une transformée de Karhunen-Loeve (voir plus bas).
- Étape 14 : construction d'un vectogramme recalé $VGM_{recalé}$, par une rotation $M(\Theta)$ du vectogramme donnant $VGM_{recalé}=M(\Theta)VGM$, de manière à faire correspondre le repère orthonormé du vectogramme recalé $VGM_{recalé}$ et celui du vectocardiogramme.
- Étape 16 : estimation du vectocardiogramme reconstruit $VCG_{reconstruit}$ à partir du vectogramme recalé, avec $VCG_{reconstruit} = W.VGM_{recalé}$.
- Étape 18 : calcul des douze dérivations ECG par application de la matrice de Dower inverse $D$, avec $ECG=D.VCG_{reconstruit}$.

On va maintenant décrire de façon plus détaillée chacune de ces étapes.

*Recueil des signaux EGM*

Il s'agit d'acquérir une pluralité de signaux endocavitaires EGM à partir d'une pluralité correspondante de dérivations correspondant à des paires d'électrodes endocavitaires reliées au boîtier de la prothèse cardiaque implantée.

Le choix des électrodes constituant ces dérivations dépend de la prothèse cardiaque considérée : stimulateur (pour le traitement des bradycardies), défibrillateur (pour le traitement des tachycardies et fibrillations) ou resynchroniseur (pour le traitement de l'insuffisance cardiaque). On distingue également trois modes de stimulation : simple, double ou triple chambre. À ces différentes fonctions correspondent des électrodes différentes, et un nombre de signaux EGM qui n'est pas le même selon le cas.

Si l'on désigne par "VD", "OD" et "VG", respectivement, les électrodes ventriculaire droite, auriculaire droite et ventriculaire gauche des sondes intracardiaques, avec "+" ou "-" indiquant la position, distale ou proximale, de l'électrode, et par "CoilV" et "SVC" les électrodes de défibrillation respectivement ventriculaire et supraventriculaire, les combinaisons d'électrodes possibles sont les suivantes (avec, à chaque fois, possibilité de définir une dérivation entre deux électrodes ou bien entre l'une des électrodes et le boîtier du générateur) :

- simple chambre : VD+, VD- (et coil V dans le cas d'un défibrillateur) ; un stimulateur simple chambre peut ainsi fournir deux signaux EGM grâce aux électrodes distale et proximale, la masse étant prise sur le boîtier. La version en défibrillateur pourra délivrer trois signaux EGM, grâce à l'électrode CoilV supplémentaire.
- double chambre : VD+, VD-, OD+, OD- (et CoilV et SVC dans le cas d'un défibrillateur) ; un stimulateur double chambre peut ainsi fournir quatre signaux EGM, et six dans la version défibrillateur.
- triple chambre : VD+, VD-, OD+, OD-, VG+, VG- (et coil V et SVC dans le cas d'un défibrillateur) ; un stimulateur triple chambre peut ainsi fournir six signaux EGM, et huit dans la version défibrillateur.

L'invention est de préférence utilisée avec des prothèses cardiaques double ou triple chambre pour pouvoir enregistrer la dépolarisation électrique dans les trois dimensions, de préférence en incluant parmi les signaux EGM sélectionnés la dérivation unipolaire VD et les dérivations unipolaires et bipolaires OD.

*Construction du vectogramme VGM*

Il s'agit (étape 12 de la figure 2) de construire une base orthogonale à partir d'un ensemble de signaux d'électrogramme. Pour la mise en oeuvre de l'invention, parmi plusieurs algorithmes possibles il est avantageux d'utiliser la transformée discrète de Karhunen-Loeve (KLT).

Le principe de l'algorithme de Karhunen-Loeve est le suivant.

Soit $X_{EGM}$ la matrice dont les $N$ composantes $EGM_1$ à $EGM_N$ sont les vecteurs d'électrogramme correspondant aux battements isolés recueillis sur les électrodes endocavitaires (pour faciliter la lecture, on a volontairement omis l'indice temporel $t$).

Les signaux $EGM_i$ sont normalisés par rapport au signal de plus forte énergie et de durée approximativement égale à la durée de l'onde à reconstruire.

On peut alors définir la matrice de covariance réelle et symétrique $C_{X_{EGM}X_{EGM}}$ , soit :

$$C_{X_{EGM}X_{EGM}} = E(X_{EGM}X_{EGM}{}^T)$$

et sa matrice de vecteurs propres $A$ associée telle que :

$$C_{X_{EGM}X_{EGM}}A = \Lambda A \ ,$$

où la matrice $A$ est constituée des $N$ valeurs propres $\lambda_i$ de $C_{X_{EGM}X_{EGM}}$.

La transformation donnée par :

$$Y = AX_{EGM} = [Y_1..Y_i..Y_N]^T$$

s'appelle la transformée discrète de Karhunen-Loeve (KLT). Sa matrice de covariance $C_{YY}$ est:

$$C_{YY} = E\left(YY^T\right) = E(AX_{EGM}X_{EGM}^T A^T) = AC_{X_{EGM}X_{EGM}} A^T$$

La matrice $C_{YY}$ a donc les mêmes valeurs propres que la matrice $C_{X_{EGM}X_{EGM}}$ , et les vecteurs $Y_i$ sont représentés dans un repère orthogonal dont les axes sont les axes principaux des vecteurs $EGM_i$ (les vecteurs propres de $C_{X_{EGM}X_{EGM}}$) et constitue le vectogramme reconstruit, que l'on notera par la suite *VGM*.

L'expérimentation montre qu'en appliquant une transformation de Karhunen-Loeve sur l'ensemble des signaux EGM recueillis, trois composantes contiennent plus de 90% de l'information. On remarque alors que le VGM estimé *VGM* a l'allure d'une ellipse dans l'espace des trois composantes principales.

La figure 3 illustre un exemple de VGM ainsi reconstruit, pour trois battements successifs. Sur cette figure, la boucle VCG est représentée en perspective (figure 3a) et en projection sur les trois plans principaux (figures 3b à 3d), plans dont les directions sont déterminées par un algorithme d'analyse en composantes principales.

Des ajustements angulaires doivent toutefois être effectués pour que les axes des vecteurs du vectogramme et celui du vectocardiogramme aient les mêmes supports. Ceci implique de procéder (étape 14 de la figure 2) à la rotation de la base formée des composantes principales du vectogramme *VGM*.

Si l'on connait les trois angles $\theta_x$, $\theta_y$ et $\theta_z$ alors un vectogramme recalé *VGM_{recalé}* peut être calculé par :

$$VGM_{recalé} = M(\Theta).VGM$$

Où la matrice de rotation $M(\Theta)$ est définie par :

$$M(\Theta) = M_x(\theta_x).M_y(\theta_y).M_z(\theta_z)$$

avec :

$$M_x(\theta_x) = \begin{bmatrix} 1 & 0 & 0 \\ 0 & cos(\theta_x) & -sin(\theta_x) \\ 0 & sin(\theta_x) & cos(\theta_x) \end{bmatrix}$$

$$M_Y(\theta_Y) = \begin{bmatrix} 0 & 1 & 0 \\ -sin(\theta_y) & 0 & cos(\theta_y) \\ -sin(\theta_y) & 0 & cos(\theta_y) \end{bmatrix}$$

$$M_Z(\theta_Z) = \begin{bmatrix} cos(\theta_z) & -sin(\theta_z) & 0 \\ sin(\theta_z) & cos(\theta_z) & 0 \\ 0 & 0 & 1 \end{bmatrix}$$

D'un point de vue pratique, les trois angles $\theta_x$, $\theta_y$ et $\theta_z$ sont inconnus. Ils doivent être appris sur une base d'apprentissage constituée d'électrogrammes EGM et d'électrocardiogrammes ECG acquis simultanément lors de la pose de l'implant.

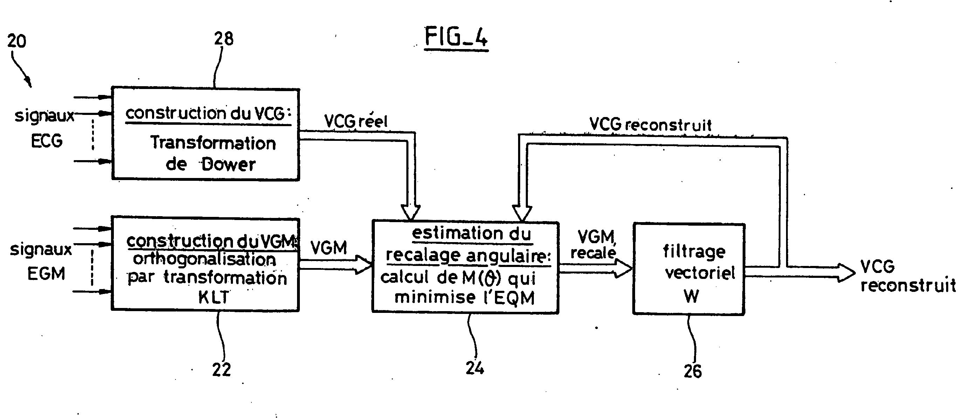

La figure 4 illustre les différentes étapes de cette phase préalable d'apprentissage permettant de définir le paramétrage du recalage angulaire. La première étape consiste à recueillir simultanément les signaux EGM et ECG correspondants (étape 20). Les signaux EGM sont utilisés pour reconstruire un VCG correspondant $VCG_{reconstruit}$, reconstruit par des étapes 22, 24 et 26 homologues des étapes 12, 14 et 16 décrites plus haut en relation avec la figure 2.

Parallèlement, les signaux ECG sont traités (étape 28) par une transformation de Dower ou analogue permettant de produire le VCG correspondant $VCG_{réel}$ directement issu des signaux ECG recueillis.

Les deux VCG, réel et reconstruit, sont alors rapprochés, et les angles $\theta_x$, $\theta_y$ et $\theta_z$ estimés de manière à minimiser au sens des moindres carrés l'erreur quadratique moyenne $e^2$:

$$\varepsilon^2 = \min_{M,\tau} \|VCG - M(\Theta).VGM_r.J_\tau\|^2$$

où $J_\tau$ permet de conserver la synchronisation temporelle des boucles vectocardiographiques.

*Estimation du vectocardiogramme VCG*

Le VCG reconstruit $VCG_{reconstruit}$ peut être alors calculé (étape 16 de la figure 2) par transformation du VGM recalé $VGM_{recalé}$, avantageusement par application d'un filtrage vectoriel non linéaire, soit :

$$VCG_{reconstruit} = [\text{VCG}_{recx}\ \text{VCG}_{recy}\ \text{VCG}_{recz}]^{T} = \text{W.VGM}_{recalé}$$

Soit encore :

$$VCG_{reconstrit} = [\text{VCG}_{recx}\ \text{VCG}_{recy}\ \text{VCG}_{recz}]^{T} = \begin{bmatrix} \text{W}_X & 0 & 0 \\ 0 & \text{W}_Y & 0 \\ 0 & 0 & \text{W}_Z \end{bmatrix} [\text{VGM}_{recx}\ \text{VGM}_{recy}\ \text{VGM}_{recz}]^{T}$$

La matrice W peut être approchée par un réseau de neurones par exemple du même type que celui décrit dans le US-A-5 740 811 (Hedberg et al.) précité.

Ce réseau de neurones est paramétré lors de la même phase d'apprentissage que celle ayant servi à déterminer les paramètres du recalage angulaire à appliquer au VGM.

Un réseau de neurones par exemple de type *"spiking"* peut réaliser les fonctions représentées en 24 et 26 après un apprentissage en essayant de minimiser au sens des moindres carrés l'erreur quadratique moyenne $e^2$. Un tel réseau est par exemple décrit par Rom et al., Adaptative Cardiac Resynchronization Therapy Device Based on Spiking Neurons Architecture with Reinforcement Learning Scheme, Classical Conditioning and Synaptic Plasticity, PACE 2005; 28: 1168-1173, Nov. 2005.

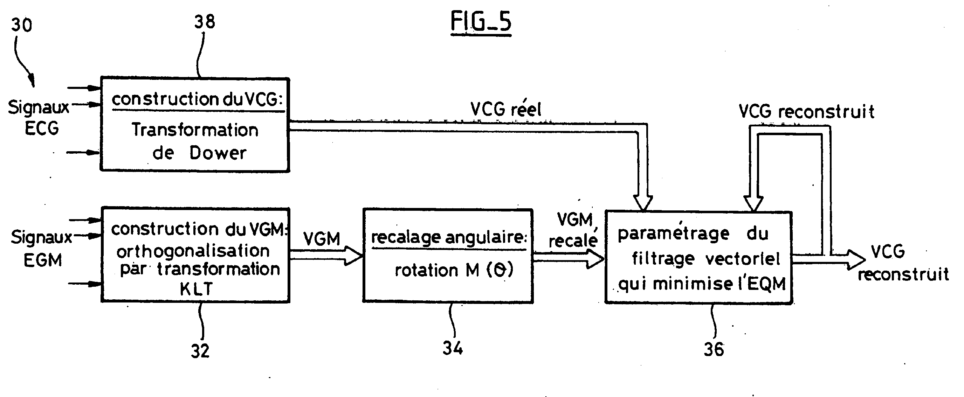

Ce paramétrage est illustré sur la figure 5. La première étape consiste à recueillir simultanément les signaux EGM et ECG correspondants (étape 30). Les signaux EGM sont utilisés pour reconstruire un VCG correspondant $VCG_{reconstruit}$, reconstruit par des étapes 32, 34 et 36 homologues des étapes 12, 14 et 16 décrites plus haut en relation avec la figure 2. Parallèlement, les signaux ECG sont traités (étape 38) par une transformation de Dower ou analogue permettant de produire le VCG correspondant $VCG_{réel}$ directement issu des signaux ECG recueillis. Les deux VCG, réel et reconstruit, sont alors rapprochés, et les paramètres du filtrage 36 sont estimés de manière à minimiser, au sens des moindres carrés, l'erreur quadratique moyenne.

Après cette phase d'apprentissage, les matrices M($\Theta$) et W sont fixées, mais peuvent aussi être mises à jour par le clinicien si ce dernier le souhaite.

Un réseau de neurones par exemple de type *"spiking"* peut également réaliser les fonctions représentées en 34 et 36, après un apprentissage en essayant de minimiser au sens des moindres carrés l'erreur quadratique moyenne $e^2$.

*Estimation des signaux ECG*

Les douze dérivations de l'ECG de surface $ECG_{reconstruit}$ sont estimées (étape 18 de la figure 2) selon le principe défini par Dower, c'est-à-dire en appliquant la matrice de Dower inverse D telle que :

$$ECG_{reconstruit} = D.VCG_{reconstruit}$$

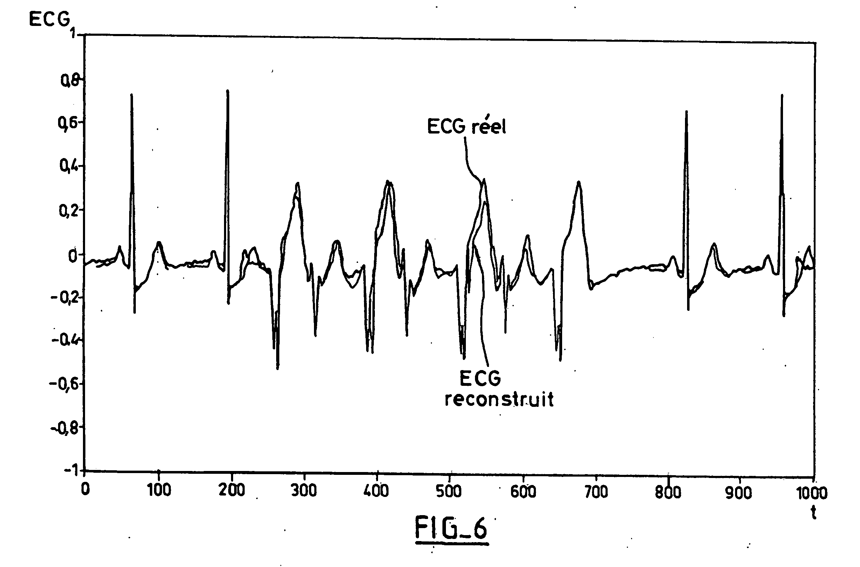

La figure 6 donne une représentation superposée d'un exemple d'ECG reconstruit à partir des signaux EGM en exploitant un réseau de neurones selon l'invention, comparé à l'ECG réel directement mesuré par des électrodes placées sur le corps du patient.

La reconstruction de l'ECG par le dispositif de l'invention permet une très bonne approche de l'ECG réel, même pour des battements cardiaques de morphologies très différentes, à la différence notamment des techniques de reconstruction d'ECG par filtrage linéaire telles que celles exposées dans le US-A-6 980 850 (Kroll et al.) précité, qui aboutit à des résultats non satisfaisants dans le cas de morphologies atypiques du signal cardiaque.

Le dispositif de reconstruction de l'ECG selon l'invention peut être le cas échéant appliquée séparément et indépendamment aux ondes P, QRS et

T du signal cardiaque, de manière à permettre une analyse spécifique.

Enfin, comme cela est illustré sur la figure 2 par les flèches en tiretés aboutissant au bloc 16, le filtrage vectoriel permettant de reconstruire le VCG à partir du VGM peut recevoir en entrée des paramètres supplémentaires susceptibles de modifier la propagation électrique de l'activité cardiaque entre le myocarde et la surface du corps du patient, tels que : position des électrodes, phase du cycle respiratoire, et/ou variations du volume thoracique (mesuré par une impédance transthoracique).

**Revendications**

1. Un dispositif médical actif comprenant des moyens de traitement de signaux représentatifs de potentiels cardiaques de dépolarisation du myocarde, ces signaux étant des signaux recueillis par une pluralité d'électrodes endocavitaires d'un dispositif médical implantable actif du type prothèse cardiaque de stimulation, resynchronisation, cardioversion et/ou défibrillation, **caractérisé en ce qu'**il comprend des moyens aptes à exécuter les étapes suivantes :

   a) acquisition (10) d'une pluralité de signaux d'électrogramme endocavitaire (EGM) sur une pluralité de dérivations intracardiaques ou intra-vasculaires, définies à partir desdites électrodes endocavitaires :
   b) calcul (12), par combinaison des signaux d'électrogramme endocavitaire (EGM) acquis à l'étape a), du vectogramme endocavitaire (VGM) correspondant ;
   d) estimation (16), à partir du vectogramme endocavitaire (VGM) calculé à l'étape b), d'un vectocardiogramme de surface reconstruit ($VCG_{re\text{-}construit}$) ; et
   e) calcul (18) des signaux d'électrocardiogramme de surface (ECG) correspondant audit vectocardiogramme de surface reconstruit ($VCG_{re\text{-}construit}$).

2. Le dispositif de la revendication 1, dans lequel l'étape b) de calcul (12) du vectogramme endocavitaire (VGM) comprend un traitement d'orthogonalisation.

3. Le dispositif de la revendication 2, dans lequel le traitement d'orthogonalisation de l'étape b) comprend une transformation de Karhunen-Loeve.

4. Le dispositif de la revendication 1, comprenant en outre, entre les étapes b) et d), une étape de : c) recalage angulaire (14) du repère orthonormé du vectogramme endocavitaire (VGM) sur celui du vectocardiogramme de surface (VCG).

5. Le dispositif de la revendication 4, comprenant en outre des moyens aptes à exécuter une phase préalable de calibration comportant une étape de détermination des paramètres dudit recalage angulaire.

6. Le dispositif de la revendication 5, dans lequel ladite étape de détermination des paramètres de recalage angulaire comprend les sous-étapes suivantes :

   i) obtention (20) d'un jeu de données de référence par acquisition simultanée de signaux d'électrogramme endocavitaire (EGM) et de signaux d'électrocardiogramme de surface (ECG) ;
   ii) calcul (28), par combinaison des signaux d'électrocardiogramme de surface (ECG) acquis à l'étape i), du vectocardiogramme de surface ($VCG_{réel}$) correspondant ;
   iii) calcul (22), par combinaison des signaux d'électrogramme endocavitaire (EGM) acquis à l'étape i), du vectogramme endocavitaire (VGM) correspondant ;
   iv) recalage angulaire (24) du repère orthonormé du hectogramme endocavitaire (VGM) sur celui du vectocardiogramme de surface ($VCG_{réel}$) ;
   v) estimation (26), à partir du vectogramme endocavitaire (VGM) calculé à l'étape iii), d'un vectocardiogramme de surface reconstruit ($VCG_{re\text{-}construit}$) ; et
   vi) ajustement (24) des paramètres du recalage angulaire de l'étape iv) pour minimiser l'écart entre le vectocardiogramme de surface ($VCG_{réel}$) calculé à l'étape ii) et le vectocardiogramme de surface reconstruit ($VCG_{reconstruit}$) calculé à l'étape v).

7. Le dispositif de la revendication 6, dans lequel ledit écart à minimiser est l'écart quadratique moyen.

8. Le dispositif de la revendication 6, dans lequel ladite étape de détermination des paramètres de recalage angulaire est mise en oeuvre par un réseau neuronal adaptatif.

9. Le dispositif de la revendication 1, dans lequel, à l'étape d), l'estimation (16) du vectocardiogramme de surface reconstruit ($VCG_{reconstruit}$) comprend un filtrage non linéaire appliqué au vectogramme endocavitaire (VGM) calculé à l'étape b).

10. Le dispositif de la revendication 9, dans lequel ledit filtrage non linéaire est mis en oeuvre par un réseau neuronal adaptatif.

11. Le dispositif de la revendication 9, comprenant en outre des moyens aptes à exécuter une phase préalable de calibration comportant une étape de détermination des paramètres dudit filtrage non linéaire.

12. Le dispositif de la revendication 11, dans lequel ladite étape de détermination des paramètres du filtrage non linéaire comprend les sous-étapes suivantes :

i) obtention (30) d'un jeu de données de référence par acquisition simultanée de signaux d'électrogramme endocavitaire (EGM) et de signaux d'électrocardiogramme de surface (ECG);

ii) calcul (38), par combinaison des signaux d'électrocardiogramme de surface (ECG) acquis à l'étape i), du vectocardiogramme de surface (VCG$_{réel}$) correspondant ;

iii) calcul (32), par combinaison des signaux d'électrogramme endocavitaire (EGM) acquis à l'étape i), du vectogramme endocavitaire (VGM) correspondant ;

iv) recalage angulaire (34) éventuel du repère orthonormé du vectogramme endocavitaire (VGM) sur celui du vectocardiogramme de surface (VCG$_{réel}$) ;

v) estimation (36), par application d'un filtrage non linéaire au vectogramme endocavitaire (VGM) calculé à l'étape iii), d'un vectocardiogramme de surface reconstruit (VCG$_{recontruit}$); et

vi) ajustement (36) des paramètres du filtrage non linéaire de l'étape v) pour minimiser l'écart entre le vectocardiogramme de surface (VCG$_{réel}$) calculé à l'étape ii) et le vectocardiogramme de surface reconstruit (VCG$_{reconstruit}$) calculé à l'étape v).

13. Le dispositif la revendication 9, dans lequel ledit filtrage non linéaire reçoit également en entrée au moins l'un des paramètres parmi : un signal respiratoire ; une information de position des électrodes endocavitaires de recueil ; la phase P, QRS, ou T du cycle cardiaque ; un signal d'impédance intracardiaque.

14. Le dispositif la revendication 1, dans lequel lesdites dérivations intracardiaques sont définies à partir d'une pluralité d'électrodes choisies parmi : électrode ventriculaire droite distale et/ou proximale, électrode auriculaire droite distale et/ou proximale, électrode ventriculaire gauche distale et/ou proximale, bobine de défibrillation ventriculaire ou auriculaire, bobine de défibrillation supra-ventriculaire.

15. Le dispositif de la revendication 1, où ledit dispositif est un dispositif implantable du type prothèse cardiaque de stimulation, resynchronisation, cardioversion et/ou défibrillation.

16. Le dispositif de la revendication 1, où ledit dispositif est un programmateur externe comprenant des moyens de téléchargement et d'analyse des signaux cardiaques recueillis et mémorisés par un implant.

17. Le dispositif de la revendication 1, où ledit dispositif est un moniteur de *home monitoring,* comprenant des moyens de téléchargement et d'analyse des signaux cardiaques recueillis et mémorisés par un implant, ainsi que des moyens de télétransmission automatique à un site distant des données ainsi recueillies.

18. Le dispositif de la revendication 1, où ledit dispositif est un serveur de données d'un site, recevant des données, d'un moniteur distant de *home monitoring* comprenant des moyens de téléchargement des signaux cardiaques recueillis et mémorisés par un implant, ainsi que des moyens de télétransmission automatique audit site des données ainsi recueillies.

**Claims**

1. An active medical device including means for processing signals representative of cardiac myocardium depolarisation potentials, said signals being signals collected by a plurality of endocardial electrodes of an active implantable medical device being of the cardiac prosthesis type for stimulation, resynchronisation, cardioversion and/or defibrillation, **characterised by** comprising means adapted to perform the following steps:

a) sensing (10) a plurality of endocardial electrogram signals (EGM) over a plurality of endocardial or intravascular derivations, defined from said endocardial electrodes;

b) calculating (12), by combining the endocardial electrogram signals (EGM) sensed at step a), the corresponding endocardial vectogram (VGM);

d) estimating (16), from the endocardial vectogram (VGM) calculated at step b), a reconstructed surface vectocardiogram (VCG$_{reconstructed}$); and

e) calculating (18) the surface electrocardiogram signals (ECG) corresponding to said reconstructed surface vectocardiogram ($VCG_{reconstruted}$).

2. The device of claim 1, wherein step b) of calculating (12) the endocardial vectogram (VGM) comprises an orthogonalization process.

3. The device of claim 2, wherein performing said orthogonalization process comprises a Karhunen-Loeve transform.

4. The device of claim 1, further comprising, between steps b) and d), a step of:

   c) angular rescaling (14) of the orthonormal coordinate system of the endocardial vectogram (VGM) upon that of the surface vectocardiogram (VCG).

5. The device of claim 4, further comprising means adapted to perform a preliminary calibration step comprising a step of determination of the parameters of said angular resealing.

6. The device of claim 5, wherein said step of determination of the parameters of said angular rescaling comprises the following sub-steps:

   (i) obtaining (20) a set of reference data through simultaneous acquisition of endocardial electrogram signals (EGM) and surface electrocardiogram signals (ECG);
   (ii) calculating (28), by combining the surface electrocardiogram signals (ECG) acquired at step i), the corresponding surface vectocardiogram ($VCG_{real}$);
   (iii) calculating (22), by combining the endocardial electrogram signals (EGM) acquired at step i), the corresponding endocardial vectogram (VGM);
   (iv) angular rescaling (24) of the orthonormal coordinate system of the endocardial vectogram (VGM) upon that of the surface vectocardiogram ($VCG_{real}$):
   (v) estimating (26), from the endocardial vectogram (VGM) calculated at step iii), a reconstructed surface vectocardiogram ($VCG_{reconstructed}$); and
   (vi) adjusting (24) the angular resealing parameters of step iv) so as to minimise the deviation between the surface vectocardiogram ($VCG_{real}$) calculated at step ii) and the reconstructed surface vectocardiogram ($VCG_{reconstructed}$) calculated at step v).

7. The device of claim 6, wherein said deviation to be minimised is the root-mean-square deviation.

8. The device of claim 6, wherein said step of determination of the parameters of said angular resealing is implemented by an adaptive neural network.

9. The device of claim 1, wherein, at step d), the estimation (16) of the reconstructed surface vectocardiogram ($VCG_{reconstructed)}$ comprises a non-linear filtering applied to the endocardial vectogram calculated at step b).

10. The device of claim 9, wherein said non-linear filtering is implemented by an adaptive neural network.

11. The device of claim 9, further comprising means adapted to perform a preliminary calibration phase comprising a step of determination of the parameters of said non-linear filtering.

12. The device of claim 11, wherein said step of determination of the parameters of said non-linear filtering comprises the following sub-steps:

   (i) obtaining (30) a set of reference data through simultaneous acquisition of endocardial electrogram signals (EGM) and surface electrocardiogram signals (ECG);
   (ii) calculating (38), by combining the surface electrocardiogram signals (ECG) acquired at step i), the corresponding surface vectocardiogram ($VCG_{real}$);
   (iii) calculating (32), by combining the endocardial electrogram signals (EGM) acquired at step i), the corresponding endocardial vectogram (VGM);
   (iv) optional angular rescaling (34) of the orthonormal coordinate system of the endocardial vectogram (VGM) upon that of the surface vectocardiogram ($VCG_{real}$);
   (v) estimating (36), through application of a non-linear filtering to the endocardial vectogram (VGM) calculated at step iii), a reconstructed surface vectocardiogram ($VCG_{reconstructed}$); and

(vi) adjusting (36) the parameters of the non-linear filtering of step v) so as to minimise the deviation between the surface vectocardiogram ($VCG_{real}$) calculated at step ii) and the reconstructed surface vectocardiogram ($VCG_{reconstructed}$) calculated at step v).

13. The device of claim 9, wherein said non-linear filtering further receives as an input at least one of the parameters from: a respiratory signal; a position information about the endocardial sensing electrodes; the P, QRS or T phase of the cardiac cycle; and an intracardiac impedance signal.

14. The device of claim 1, wherein said intracardiac derivations are defined from a plurality of electrodes chosen among: right ventricular distal and/or proximal electrode, right atrial distal and/or proximal electrode, left ventricular distal and/or proximal electrode, ventricular or atrial defibrillation coil, and supra-ventricular defibrillation coil.

15. The device of claim 1, wherein said device is an implantable device of the cardiac prosthesis type for stimulation, resynchronisation, cardioversion and/or defibrillation.

16. The device of claim 1, wherein said device is an external programmer including means for downloading and analysing the cardiac signals sensed and stored by an implant.

17. The device of claim 1, wherein said device is a home monitoring monitor, including means for downloading and analysing the cardiac signals sensed and stored by an implant, as well as means for automatic remote transmission to a distant site of data thus collected.

18. The device of claim 1, wherein said device is a data server of a remote site, receiving data from a remotely located home monitoring monitor including means for downloading the cardiac signals sensed and stored by an implant, as well as means for automatic remote transmission to said site of data thus collected.

**Patentansprüche**

1. Aktive medizinische Vorrichtung, die Mittel aufweist zum Verarbeiten von Signalen, die für Herzpotentiale der Depolarisation des Herzmuskels repräsentativ sind, wobei diese Signale Signale sind, die von einer Vielzahl von endokavitären Elektroden einer aktiven implantierbaren medizinischen Vorrichtungen vom Typ Herzprothese zur Stimulation, Resynchronisation, Kardioversion und/oder Defibrillation aufgenommen werden, die **dadurch** charakterisiert ist, dass sie Mittel aufweist, die dazu geeignet sind die folgenden Schritte auszuführen:

   a) Erfassung (10) einer Vielzahl an endokavitären Elektrogrammsignalen (EGM) über eine Vielzahl an intrakardialen oder intravaskulären Ableitungen, die ausgehend von den endokavitären Elektroden bestimmt werden;
   b) Berechnung (12) des entsprechenden endokavitären Vektogramms (VGM) durch Kombination der endokavitären Elektrogrammsignale (EGM), die in Schritt a) erfasst wurden;
   d) Schätzung (16) eines rekonstruierten Vektrokardiogramms der Oberfläche ($VCG_{reconstruit}$) ausgehend von dem endokavitären Vektogramm (VGM), das in Schritt b) berechnet wurde; und
   e) Berechnung (18) von Signalen des Elektrokardiogramms der Oberfläche (ECG), das dem rekonstruierten Vektokardiogramm der Oberfläche ($VCG_{reconstruit}$) entspricht.

2. Vorrichtung nach Anspruch 1, in welcher der Schritt b) der Berechnung (12) des endokavitären Vektogramms (VGM) eine Orthogonalisierungsverarbeitung umfasst.

3. Vorrichtung nach Anspruch 2, in welcher die Orthogonalisierungsverarbeitung des Schrittes b) eine Karhunen-Loeve Transformation umfasst.

4. Vorrichtung nach Anspruch 1, die darüber hinaus zwischen Schritten b) und d) einen Schritt aufweist zur:

   c) Winkeländerung (14) des orthonormiertcn Koordinatensystems des endokavitären Vektogramms (VGM) auf jenes des Vektokardiogramms der Obefläche (VCG).

5. Vorrichtung nach Anspruch 4, weiter aufweisend Mittel, die dazu geeignetsind eine vorhergehende Phase der Kalibrierung auszuführen, die einen Schritt der Bestimmung der Parameter der Winkeländerung aufweist.

6. Vorrichtung nach Anspruch 5 in welcher der Schritt der Bestimmung der Parameter der Winkeländerung die folgenden Unterschritte aufweist:

i) Erhalt (20) eines Referenzdatensatzes durch gleichzeitiges Erfassen von endokavitären Elektrogrammsignalen (EGM) und Signalen des Elektrokardiogramms der Oberfläche (ECG);
ii) Berechnung (28) des entsprechenden Vektokardiogramms der Oberfläche ($VCG_{réel}$) durch Kombination von Signalen des Elektrokardiogramms der Oberfläche (ECG), die in Schritt i) erfasst wurden;
iii) Berechnung (22) des entsprechenden endokavitären Vektogramms (VGM) durch Kombination von Signalen des endokavitären Elektrogramms (EGM), die in Schritt i) erfasst wurden;
iv) Winkeländerung (24) des orthonormiertcn Koordinatensystems des endokavitären Vektogramms (VGM) auf jenes des Vektokardiogramms der Oberfläche ($VCG_{réel}$);
v) Schätzung (26) eines rekonstruierten Vektokardiogramms der Oberfläche ($VCG_{reconstruit}$) ausgehend von dem endokavitären Vektogramm (VGM), das in Schritt iii) berechnet wurde; und
vi) Anpassung (24) der Parameter der Winkelverschiebung des Schrittes iv) um die Abweichung zwischen dem Vektokardiogramm der Oberfläche ($VCG_{réel}$), das in Schritt ii) berechnet wurde, und dem rekonstruierten Vektokardiogramm der Oberfläche ($VCG_{reconstruit}$), das in Schritt v) berechnet wurde, zu minimieren.

7. Vorrichtung nach Anspruch 6, in welcher die zu minimierende Abweichung die mittlere quadratische Abweichung ist.

8. Vorrichtung nach Anspruch 6, in welcher der Schritt der Bestimmung der Parameter der Winkeländerung durch ein adaptives neuronales Netzwerk ausgerührt wird.

9. Vorrichtung nach Anspruch 1, in welcher in Schritt d), die Schätzung (16) des rekonstruierten Vektokardiogramms der Oberfläche ($VCG_{reconstruit}$) eine nicht-lineare Filterung umfasst, die auf das endokavitäre Vektogramm (VGM), das in Schritt b) berechnet wurde, angewandt wird.

10. Vorrichtung nach Anspruch 9, in welcher die nicht-lineare Filterung von einem adaptiven neuronalen Netz ausgeführt wird.

11. Vorrichtung nach Anspruch 9, weiter aufweisend Mittel, die dazu geeignet sind eine vorhergehende Phase der Kalibrierung auszuführen, die einen Schritt der Bestimmung der Parameter der nicht-linearen Filterung umfasst.

12. Vorrichtung nach Anspruch 11, in welcher der Schritt der Bestimmung der Parameter der nicht-linearen Filterung die folgenden Unterschritte umfasst:

i) Erhalt (30) eines Referenzdatensatzes durch gleichzeitiges Erfassen von endokavitären Elektrogrammsignalen (EGM) und Signalen des Elektrokardiogramms der Oberfläche (ECG);
ii) Berechnung (38) des entsprechenden Vektokaridogramms der Oberfläche ($VCG_{réel}$) durch Kombination von Signalen des Elektrokardiogramms der Oberfläche (ECG), die in Schritt i) erfasst wurden;
iii) Berechnung (32) des entsprechenden endokavitären Vektogramms (VGM) durch Kombination von endokavitären Elektrogrammsignalen (EGM), die in Schritt i) erfasst wurden;
iv) eventuell Winkeländerung (34) des orthonormierten Koordinatensystems des endokavitären Vektogramms (VGM) auf jenes des Vektokardiogramms der Overfläche ($VCG_{réel}$);
v) Schätzung (36) eines rekonstruierten Vektokardiogramms der Oberfläche ($VCG_{reconstruit}$) durch Anwendung einer nicht-linearen Filterung auf das endokavitäre Vektogramm (VGM), das in Schritt iii) berechnet wurde; und
vi) Anpassung (36) der Parameter der nicht-linearen Filterung des Schrittes v) um die Abweichung zwischen dem Vektokardiogramm der Oberfläche ($VCG_{réel}$), das in Schritt ii) berechnet wurde, und dem rekonstruierten Vektokardiogramm der Oberfläche ($VCG_{reconstruit}$), das in Schritt v) berechnet wurde, zu minimieren,

13. Vorrichtung nach Anspruch 9, in welcher die nicht-lineare Filterung ebenso am Eingang wenigstens einen der Parameter aufnimmt aus: ein Atemsignal, eine Positionsinformation der endokavitären Aufnahmeelektroden; der Phase P, QRS oder T des Herzzyklus; ein intrakardiales Impedanzsignal.

14. Vorrichtung nach Anspruch 1, in welcher die intrakardialen Ableitungen ausgehend von einer Vielzahl an Elektroden bestimmt werden, die gewählt werden aus: entfernte und/oder nahe rechte ventrikuläre Elektrode, entfernte und/odcr nahe rechte aurikuläre Elektrode, entfernte und/oder nahe linke ventrikuläre Elektrode, Spule zur ventrikulären oder aurikulären Defibrillation, Spule zur supra-ventrikulären Defibrillation.

**15.** Vorrichtung nach Anspruch 1, in der die Vorrichtung eine implantierbare Vorrichtung des Typs Herzprothese zur Stimulation, Resynchronisation, Kardioversion und/oder Defibrillation ist.

**16.** Vorrichtung nach Anspruch 1, in der die Vorrichtung ein externer Programmierer ist, der Mittel zum Herunterladen und zur Analyse von Herzsignalen, die von einem Implantat aufgenommen wurden und gespeichert wurden, aufweist.

**17.** Vorrichtung nach Anspruch 1, in der die Vorrichtung ein *home monitoring* Überwacher ist, die Mittel zum Herunterladen und zur Analyse von Herzsignalen, die von einem Implantat aufgenommen wurden und gespeichert wurden, sowie Mittel zur automatischen Fernübermittlung der so aufgenommenen Daten an eine entfernte Stelle aufweist.

**18.** Vorrichtung nach Anspruch 1, in der die Vorrichtung ein Datenserver einer Stelle ist, die Daten eines entfernten *home monitoring* Überwachers empfängt, der Mittel zum Herunterladen von Herzsignalen, die von einem Implantat aufgenommen wurden und gespeichert wurden, sowie Mittel zur automatischen Fernübermittlung von so aufgenommenen Daten an die Stelle aufweist.

## FIG_1

(b)

**plan frontal (XY)**

(c)

**plan sagittal (YZ)**

**plan horizontal (XZ)**

VCG

(d)

(a)

## FIG_3

(b)

(c)

VGM

(d)

(a)

## FIG_2

signaux EGM **10** →

| construction du VGM: orthogonalisation par transformation KLT **12** | →VGM→ | recalage angulaire: rotation M(θ) **14** volume | →VGM recalé→ | filtrage vectoriel W **16** | →VCG reconstruit→ | transformation de Dower inverse **18** | → ECG reconstruit (12 dérivations |

respiration thoracique | position des électrodes

phase du cycle cardiaque

EP 1 902 750 B1

FIG_4

FIG_5

30

38

Signaux → ECG

| construction du VCG: |
| Transformation de Dower |

VCG réel

VCG reconstruit

Signaux → EGM

| construction du VGM: orthogonalisation par transformation KLT |

VGM

| recalage angulaire: rotation M (θ) |

VGM recalé

| paramétrage du filtrage vectoriel qui minimise l'EQM |

VCG reconstruit

32

34

36

EP 1 902 750 B1

EP 1 902 750 B1

ECG réel

ECG reconstruit

FIG_6

19

**EP 1 902 750 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5740811 A, Hedberg
- US 6980850 A, Kroll
- US 20050288600 A, Zhang
- US 4850370 A, Dower

### Littérature non-brevet citée dans la description

- **E. FRANK.** An Accurate, Clinically Practical System for Spatial Vectocardiography. *Circulation,* Mai 1956, vol. 13, 737-749
- **ROM et al.** Adaptative Cardiac Resynchronization Therapy Device Based on Spiking Neurons Architecture with Reinforcement Learning Scheme, Classical Conditioning and Synaptic Plasticity. *PACE 2005,* Novembre 2005, vol. 28, 1168-1173